# EUROPEAN PATENT SPECIFICATION

(11) **EP 4 356 128 B1**
(45) Date of publication and mention of the grant of the patent: **04.02.2026**
(21) Application number: 22734571.7
(22) Date of filing: 15.06.2022
(51) Int. Cl.: G01N 33/52, G01N 33/543, G01N 33/58

(54) **METHODS FOR DETECTING AN ANALYTE USING STRUCTURE SWITCHING BINDING AGENTS**
VERFAHREN ZUM NACHWEIS EINES ANALYTEN UNTER VERWENDUNG STRUKTURWECHSELNDER BINDEMITTEL
PROCÉDÉS DE DÉTECTION D'UN ANALYTE A L'AIDE D'AGENTS DE LIAISON A COMMUTATION DE STRUCTURE

(30) Priority: 17.06.2021 US 202117350822
(43) Date of publication of application: 24.04.2024
(73) Proprietor: F. Hoffmann-La Roche AG, 4070 Basel (CH); Roche Diagnostics GmbH, 68305 Mannheim (DE)
(72) Inventor: ASTIER, Yann, Pleasanton, California 94588 (US); HEINDL, Dieter, 82377 Penzberg (DE); KUCHELMEISTER, Hannes, 82377 Penzberg (DE); STENGELE, Nikolaus-Peter, 82377 Penzberg (DE)
(74) Representative: Altmann Stößel Dick Patentanwälte PartG mbB
(86) International application number: PCT/EP2022/066342
(87) International publication number: WO 2022/263529

(56) References cited:
- EP-A1- 3 252 461
- WO-A1-2009/039437
- WO-A2-2007/092909
- US-A1- 2014 266 186

## Description

### FIELD OF THE INVENTION

The present invention relates to diagnostic test and technology. In particular, it relates to a method for determining an analyte suspected to be present in a sample comprising contacting said sample with at least one sensor element comprising at least one binding agent which is capable of specifically binding to the analyte and which comprises at least one magnetic label; and in functional proximity thereto a magnetic tunnel junction generating a signal which is altered upon binding of the analyte to the binding agent for a time and under conditions which allow for specific binding of the analyte suspected to be present in the sample to the at least one binding agent, measuring an altered signal generated by the magnetic tunnel junction upon analyte binding to the at least one binding agent comprising the at least one magnetic label, and determining the analyte based on the altered signal which is generated by the magnetic tunnel junction.

### BACKGROUND OF THE INVENTION

Immunoassays are widely used for various diagnostic purposes. Several setups have been developed for immunoassays. One of the most popular immunoassays is the enzyme-linked sorbent immunoassay (ELISA).

In ELISA, a liquid sample containing an anylte of interest or suspected to contain an anlyte of interest is applied onto a stationary solid phase with special binding properties due to the presence of antibodies or antibody-like molecules such as aptamers. After sample application, multiple reagents are sequentially added, incubated, and washed away in order to carry out and stop an anytical detection reaction. After carrying out all these steps, physical or chemical properties in the setup, usually the liquid phase, are changed that can be detected. Typically, optical change such as color development by the product of an enzymatic reaction will happen in the final liquid phase. These changes correlate to the presence or abundance of the analyte of interest present in the investigated sample. The typically quantitative read-out is usually based on detection of intensity of transmitted light by spectrophotometry, which involves quantitation of transmission of some specific wavelength of light through the liquid. The sensitivity of detection depends on amplification of the signal during the analytic reactions. Since enzyme reactions are very well known amplification processes, the signal is generated by enzymes which are linked to the detection reagents in fixed proportions to allow accurate quantification.

For an ELISA setup, the analyte binding agent, e.g., an antibody, is immobilized on a solid phase such as a solid support structure. Usually, the antibody is coated and dried onto the transparent bottom and sometimes also side wall of a well in an analytic multi-well plate or in an analytic vial. However, nanoparticles or other beads can also be used as solid phases for ELISAs.

For research and diagnostic, ELISAs are often used in the format of so-called sandwich ELISAs. In a sandwich format, an immobilized capture antibody is used to capture, i.e. specifically bind to, an analyte present in a sample applied to said immobilized antibody. After the capture antibody has specifically bound to the analyte, the sample material is washed away. In a subsequent step the analyte bound to the immobilized antibody is incubated with a detection antibody that specifically binds to the analyte or the complex of analyte and capture antibody. The detection antibody typically contains a detectable linker or an adaptor molecule which allows for attracting such detectable labels from a solution.

However, in light of the fragile immune complexes which are required for signal generation and the various components used for such an ELISA in the sandwich format, there are many possibilities for undesired binding of detection antibodies and thus for the generation of false positive signals. Therefore, sandwich ELISAs used for research often need validation because of the risk of false positive results. Moreover, there are limitations with respect to sensitivity and specificity due to the various drawbacks of such a fragile multi-component assay format.

Document WO 2007/092909 discloses a method of detecting a target binding molecule using a magnetoresistive molecular interaction sensor.

### SUMMARY OF THE INVENTION

The present invention is defined in the appended claims, and relates to a method for determining an analyte suspected to be present in a sample comprising:
(a) contacting said sample with at least one sensor element comprising:
   (i) at least one binding agent which is capable of specifically binding to the analyte and which comprises at least one magnetic label; and in functional proximity thereto
   (ii) a magnetic tunnel junction generating a signal which is altered upon binding of the analyte to the binding agent
   for a time and under conditions which allow for specific binding of the analyte suspected to be present in the sample to the at least one binding agent;
(b) measuring an altered signal generated by the magnetic tunnel junction upon analyte binding to the at least one binding agent comprising the at least one magnetic label; and
(c) determining the analyte based on the altered signal which is generated by the magnetic tunnel junction.

The binding agent is an aptamer or fragment thereof.

Described herein is also a device for determining an analyte suspected to be present in a sample comprising at least one sensor element comprising:
(i) at least one binding agent which is capable of specifically binding to the analyte and which comprises at least one magnetic label; and in functional proximity thereto
(ii) a magnetic tunnel junction generating a signal which is altered upon binding of the analyte to the binding agent.

Described herein is also an aptamer which is capable of specifically binding to an analyte and which comprises at least one magnetic label, said aptamer being capable of altering its structure upon binding of the analyte such that the position of the at least one magnetic label is altered.

The present description also contemplates a method for identifying an aptamer as defined before comprising the steps of:
a) providing an aptamer library;
b) identifying a plurality of aptamer candidates by an iterative selection-amplification process which are capable of specifically binding to an analyte of interest;
c) synthesizing said aptamer candidates in an array format such that each aptamer candidate is capable of regulating the signal elicited by a sensor element, preferably, a magnetic tunnel junction, linked to the aptamer candidate;
d) attaching at least one magnetic label to the aptamer candidate on the array;
e) contacting the array with the analyte of interest;
f) determining the response curve of the sensor elements based on the elicited signals; and
g) identifying the aptamer as defined before based on an evaluation of the individual response curves.

Also described is a kit for determining an analyte suspected to be present in a sample comprising the device or the aptamer described herein.

### FIGURES

**Figure** : Schematic view of an aptamer in an unbound stage (A) or after specific binding of the analyte to be detected (B).

### DETAILED DESCRIPTION

The present invention relates to a method for determining an analyte suspected to be present in a sample comprising:
(a) contacting said sample with at least one sensor element comprising:
   (i) at least one binding agent which is capable of specifically binding to the analyte and which comprises at least one magnetic label; and in functional proximity thereto
   (ii) a magnetic tunnel junction generating a signal which is altered upon binding of the analyte to the binding agent
   for a time and under conditions which allow for specific binding of the analyte suspected to be present in the sample to the at least one binding agent;
(b) measuring an altered signal generated by the magnetic tunnel junction upon analyte binding to the at least one binding agent comprising the at least one magnetic label; and
(c) determining the analyte based on the altered signal which is generated by the magnetic tunnel junction.

The binding agent is an aptamer or fragment thereof, as defined in appended claim 1.

It is to be understood that in the specification and in the claims, "a" or "an" can mean one or more, depending upon the context in which it is used. Thus, for example, reference to "an" item can mean that at least one item can be utilized.

As used in the following, the terms "have", "comprise" or "include" are meant to have a non-limiting meaning or a limiting meaning. Thus, having a limiting meaning these terms may refer to a situation in which, besides the feature introduced by these terms, no other features are present in an embodiment described, i.e. the terms have a limiting meaning in the sense of "consisting of" or "essentially consisting of". Having a non-limiting meaning, the terms refer to a situation where besides the feature introduced by these terms, one or more other features are present in an embodiment described.

Further, as used in the following, the terms "preferably", "more preferably", "most preferably", "particularly", "more particularly", "typically", and "more typically" or similar terms are used in conjunction with additional or alternative features, without restricting alternative possibilities.

Further, it will be understood that the term "at least one" as used herein means that one or more of the items referred to following the term may be used in accordance with the invention. For example, if the term indicates that at least one item shall be used this may be understood as one item or more than one item, i.e. two, three, four, five or any other number. Depending on the item the term refers to the skilled person understands as to what upper limit the term may refer, if any.

The method according to the present invention may consist of the aforementioned steps (a) to (c) or may comprise further steps, such as pretreating or isolating the sample prior to step (a) and/or after step (c) further one or more steps of evaluating the determined analyte, e.g., by comparing it to references in order to provide a diagnostic, prognostic, environmental-relevant, agricultural-relevant or analytically-relevant conclusion depending on the aim of the determination of the analyte.

The term "determining" as used herein encompasses any kind of qualitative or quantitative determinations of the analyte. A qualitative determination aims at determining the presence or the absence of the analyte in the sample whereas quantitative determination aims at determining the amount of the analyte. The quantitative determination, i.e. the determination of the amount, includes determining the absolute amount (e.g. the total amount by weight or number of molecules present in the sample) or a relative amount (e.g., an amount relative to the sample volume (concentration) or a classification such as a score (e.g., "high amount", "low amount" and the like). Typically, determining an analyte comprises determining the presence, absence or amount of said analyte.

The term "analyte" as referred to herein relates to any type of molecule or agent which is suitable for being determined by the method of the invention. It will be understood that such a molecule or agent may have a size and/or structure that allows binding of the first and second binding agents referred to herein. Moreover, there may be upper size limitations as well since the first and second binding agents as well as the linking agent must be capable of exert their functions as described elsewhere herein in detail. Typically, the analyte referred to herein is a biological molecule such as a protein, a peptide, a nucleic acid, e.g., a DNA or RNA or a small molecule such as a lipid or metabolite, a polyketide including, e.g., flavonoids and isoflavonoids, an isoprenoid including, e.g., terpenes, sterols, steroids, carotenoids, xanthophylls, a carbohydrate, a phenylpropanoide, an alcaloide, a benzenoide, an indole, a porphyrine, a hormone, a vitamin, a cofactor, a lignin, a glucosinolate, a purine, a pyrimidine, a nucleoside, a nucleotide, an alcohol, an alkane, an alkene, an alkine, an aromatic compound, a ketone, an aldehyde, a carboxylic acid, a ester, an amine, an imine, an amide, a cyanide, an amino acid, a thiol, a thioester, a phosphate ester, a sulfate ester, a thioether, a sulfoxide or an ether. The small molecule may be, e.g., a toxin. However, the method of the invention can also be used for determining viruses or even bacterial cells as analytes. Analytes to be determined by the present invention may also be molecules which are present in environmental samples and which may be useful as indicators for, e.g., environmental pollution, agriculture or other environmental conditions. Typically, said analyte is a protein, peptide, virus, bacterial cell or small molecule, preferably, small molecule toxin.

The term "sample" as used herein relates to any part or aliquot of a composition comprising or suspected to comprise the analyte to be determined. Such a sample may be a biological sample, typically, isolated from an organism, such as a body fluid or biopsy sample, or may be a composition comprising organism such as a cultured cells. Typically, said biological sample is investigated by the method of the invention for medical purposes, such as diagnosing or predicting a disease or medical condition. Moreover, the sample may be an environmental sample or an artificial sample. An environmental sample may be derived from any non-biological, natural source, e.g., environmentally occurring solutions such as water or from compositions such as soil. An artificial sample may be a sample obtained from an artificial source, e.g., a product composition which may be manufactured or an intermediate composition which may occur during a manufacturing process for a product. Such artificial samples may be investigated, e.g., for quality control purposes or for determining the amount of specific ingredients. Typically, said sample in accordance with the method of the present invention is a biological sample, preferably, a body fluid or biopsy sample.

The term "sensor element" as used in accordance with the present invention comprises at least one binding agent which is capable of specifically binding to the analyte and which comprises at least one magnetic label. The at least one binding agent is located in the sensor element in functional proximity to a magnetic tunnel junction. The magnetic tunnel junction shall be in functional proximity such that a signal can be altered if the at least one magnetic label of the at least one binding agent alters its position víz-a-víz the magnetic tunnel junction. Typically, the at least one binding agent may be immobilized on a solid support. A solid support as referred to herein is a solid composition of matter which can serve as a basis for immobilizing molecules and, in particular, the at least one binding agent and the magnetic tunnel junction. A solid support may comprise inorganic or organic compounds or both. Typically, suitable inorganic compounds for a solid support may be selected from the group consisting of: silica, porous glass, aluminosilicates, borosilicates, metal oxides (e.g. aluminum oxide, iron oxide, nickel oxide) and clay containing one or more of these. Alternatively, the solid support may comprise an organic compound such as a cross-linked polymer. Non-limiting examples of a suitable cross-linked polymer may be selected from the group consisting of: polyamide, polyether, polystyrene and mixtures thereof. The skilled artisan is well aware of how to select a suitable solid support based on the kind of sample to investigated, the method envisaged for detection of the analyte, the kind of magnetic label to be used for detecting the analyte and/or the kind of magnetic tunnel junction used for the sensor element.

The term "binding agent" referred to herein relates to a molecule which is capable of specifically binding to the analyte, i.e. a molecule which does not bind to and, thus, does not cross-react with other molecules than the analyte suspected to be present in the sample. Specific binding, in principle, can be tested by techniques well known in the art including screening assays for identifying agents specifically binding an analyte from libraries comprising different candidate agents.

Molecules which may be used as binding agents being capable of specifically binding a desired analyte may be antibodies. Antibodies as binding agents as meant in accordance with the present description encompass all types of antibodies which, preferably, specifically bind to the analyte. Preferably, an antibody may be a monoclonal antibody, a polyclonal antibody, a single chain antibody, a chimeric antibody or any fragment of such antibodies being still capable of specifically binding to the analyte. Such fragments comprised by the term antibody as used herein encompass a bispecific antibody, a synthetic antibody, an Fab, F(ab)2 Fv or scFv fragment or a chemically modified derivative of any of these antibody fragments. Antibodies or fragments thereof, in general, which specifically bind to a desired analyte can be obtained by using methods which are described, e.g., in Harlow and Lane "Antibodies, A Laboratory Manual", CSH Press, Cold Spring Harbor, 1988. Monoclonal antibodies can be prepared by the techniques which comprise the fusion of mouse myeloma cells to spleen cells derived from immunized mammals and, preferably, immunized mice (Köhler 1975, Nature 256, 495, and Galfré 1981, Meth. Enzymol. 73,3). The skilled person is well aware of how specific binding can be tested by techniques well known in the art, such as immunological or biochemical techniques including immunoassays, cell sorting or Western blotting or plasmon surface resonance measurements.

The molecules which are used as binding agents being capable of specifically binding a desired analyte are aptamers. An aptamer as a binding agent in accordance with the present invention may be an oligonucleic acid or peptide molecule that binds to a specific target analyte (Ellington 1990, Nature 346 (6287): 818-22). Bock 1992,

Nature 355 (6360): 564-6). Oligonucleic acid aptamers are engineered through repeated rounds of selection or the so-called systematic evolution of ligands by exponential enrichment (SELEX technology). Peptide aptamers usually comprise of a variable peptide loop attached at both ends to a protein scaffold. This double structural constraint shall increase the binding affinity of the peptide aptamer into the nanomolar range. Said variable peptide loop length is, preferably, composed of ten to twenty amino acids, and the scaffold may be any protein having improved solubility and compacity properties, such as thioredoxin-A. Peptide aptamer selection can be made using different systems including, e.g., the yeast two-hybrid system (see e.g., Hoppe-Seyler 2000, J Mol Med. 78 (8): 426-30). Also encompassed according to the present invention are any fragments of said aptamers that are still capable of specifically binding to the analyte. Said fragments can be used in isolated form or may be part of fusion molecules, i.e. molecules comprising said aptamer fragment as well as other parts such as linker moieties or adapter molecules. The skilled person is well aware of how specific binding can be tested by techniques well known in the art, such as plasmon surface resonance measurements. Preferred methods for identifying an aptamer useful in the aforementioned method of the invention are described elsewhere herein. The binding agent according to the present invention, more typically, is an aptamer identified by such a method.

Molecules which may be used as binding agents being capable of specifically binding a desired analyte may also be receptor molecules. A receptor molecule as a binding agent is, typically, a protein that specifically binds to a ligand and which become activated upon ligand binding to exert its biological function. Such a receptor molecule or a fragment thereof being still capable of specifically binding to the ligand may also be used as a binding agent for the ligand as analyte or a molecule which is derived from the ligand but still capable of being bound by the receptor molecule or fragment thereof such as antagonistically or agonistically acting variants of a ligand. Preferably, the receptor molecule envisaged as a binding agent may be a transmembrane type receptor protein, such as a G-protein coupled receptor, e.g., a metabolic receptor, an enzyme linked receptor such as a receptor tyrosine kinase, e.g., a growth factor receptor, an immunological receptor, such as a virus receptor, a cell surface antigen, a T cell receptors, e.g., CD4, CD3 or CD8, or a MHC protein, a cell adhesion molecule, such as an integrin, cadherin, selectin or syndecan, a neuronal receptor or a pathogen receptor such as Toll-like receptors. The receptor molecule may also be a nuclear receptor protein such as a nuclear hormone receptor, e.g., the glucocorticoid receptor, retinoic acid receptor or thyroid hormone receptor. The skilled person is well aware of receptor molecules or fragments thereof which specifically bind to an analyte to be determined or fragments thereof. Moreover, specific binding can be tested by techniques well known in the art, such as plasmon surface resonance measurements.

Yet, molecules which may be used as binding agents being capable of specifically binding a desired analyte may also be ligand molecules. A ligand molecule as a binding agent is, typically, a protein or peptide that specifically binds to a receptor molecule and which activates upon receptor binding the said receptor molecule. Such a ligand molecule or a fragment thereof being still capable of specifically binding to the receptor molecule may also be used as a binding agent for the receptor as analyte or a molecule which is derived from the receptor molecule but still capable of being bound by the ligand molecule or fragment thereof such as soluble variants of a receptor. Moreover, a ligand may also be any antigen which may be used to determine a certain antibody in a sample of an organism. Preferably, the ligand molecule envisaged as a binding agent may be a peptide hormone, a neurotransmitter, a growth factor, such as angiopoietin, a BMP, a neutrotrophic factor, EGF, an epiphrin, EPO, a FGF, a GDNF, a GDF, insulin or an insulin-like growth factor, a TGF, a neutrophin, a VEGF, a cytokine, such as an interleukin, interferon, lymphokine, monokine, colony stimulating factor or chemokine, a extracellular matrix protein such as fibronectin, vitronectin, collagen, ankyrin or laminin, or the like. The skilled person is well aware of ligand molecules or fragments thereof which specifically bind to an analyte to be determined or fragments thereof. Moreover, specific binding can be tested by techniques well known in the art, such as plasmon surface resonance measurements.

Further preferably, the binding agent may be a Designed Ankyrin Repeat Protein (DARPin). DARPins are genetically engineered antibody mimetic proteins which can be designed to achieve highly specific and high-affinity target protein binding. They are derived from natural ankyrin repeat proteins. Typically, DARPins comprise at least three repeat modules, of which the most N- and the most C-terminal modules (also referred to as "caps") protect the hydrophobic core of the protein (Binz 2003, Journal of Molecular Biology. 332 (2): 489-503).

Typically, said binding agent is an aptamer or fragment thereof.

The at least one binding agent according to the present invention shall elicit an altered signal in the magnetic tunnel junction once the analyte has been specifically bound thereto. This is typically achieved by a conformational change or other molecular movement resulting from said specific binding such that the position of the at least one magnetic label becomes altered and elicits the altered signal in the magnetic tunnel junction. According to the invention, said at least one binding agent is, thus, altered in its structure upon binding to the analyte such that the position of the at least one magnetic label becomes located closer (positive mode) or farther away (negative mode) in respect to the sensitivity field of the magnetic tunnel junction.

The at least one binding molecule according to the present invention may also comprise, preferably, an adaptor for immobilization. Typically, said adapter allows for immobilization of the at least one binding agent on a solid support comprised in the sensor element. Typical adaptor molecules useful in accordance with the present invention for immobilization of the binding agent are well known in the art and dependent on the nature of the binding agent as well as on the nature of the solid support used for immobilization in the sensor. More typically, such an adaptor is LNA, L-DNA or L-LNA. In such a case, more typically, the binding agent shall be an aptamer as specified elsewhere herein.

The term "magnetic label" as used herein relates to molecules which can be detected in functional proximity to a magnetic tunnel junction. Accordingly, a magnetic label is typically envisaged in accordance with the present invention as detectable label. Typical magnetic labels comprise iron-platinum nanoparticles, iron nanoparticles, nickel nanoparticles or cobalt nanoparticles. The magnetic label may be reversibly or permanently bound to the binding agent. To this end, the magnetic label may be a molecule which is capable of reversibly binding to the binding agent or it may be a molecule or moiety which is already part of the said binding agent. The binding agent may comprise the at least one magnetic label as a part of the molecule. Alternatively, the at least one magnetic label may be linked to the first binding molecule by a linker. Permanent linkage as well as reversible linkage are envisaged in accordance with the present invention. Reversible linkage may be achieved, e.g., by using biotin-streptavidin based molecular adapter systems well known in the art.

The term "magnetic tunnel junction" as used herein refers to magneto-responsive detectors such as magnetic tunnel junctions or magnetic spin valves (see, e.g., US 5,981,297; Fernandes 2020, Nanomedicine: Nanotechnology, Biology, and Medicine 30, 102287 or Denmark 2019, Journal of Electronic Materials (48): 4749-4761). The magnetic tunnel junction of the sensor element, typically, is placed on the opposite of the at least one binding agent on a solid support. Depending on the magnetic label, different detection techniques may be applied. Moreover, depending on the detection technique, it may be necessary to apply a magnetic field to the magnetic tunnel junction.

It will be understood that the detected presence, absence or amount of the magnetic label can be subsequently transmitted to an evaluation unit. Said evaluation unit may, preferably, comprise a data processing element, such as a computer, with an implemented algorithm for determining the presence absence or amount of an anaylte in the sample based on the detected presence, absence or amount of magnetic label(s).

Such an implemented algorithm may evaluate the measured altered signals elicited from the magnetic label(s) for signal strength, signal duration and other predetermined parameters. Based on said evaluation, truly positive signals can be identified and validated and noise signals can be identified and disregarded for further evaluations. The skilled artisan is well aware of what algorithms may be used and how they can be implemented. Preferably, said measuring the altered signal generated by the magnetic tunnel junction upon analyte binding to the at least one binding agent comprises measuring the strength of the generated signal and/or its duration. Moreover, said measuring, preferably, comprises measuring a change in the altered signal over time and evaluating said change, preferably, by a computer-implemented validation algorithm. Such validation algorithms, typically, comprise artificial intelligence algorithms, machine learning algorithms and the like.

In step (a) according to the method of the preset invention, a sample which comprises the analyte to be determined or which is suspected to comprise the analyte to be determined is brought into contact with a sensor element as specified elsewhere herein in detail.

The term "contacting" as used herein relates to bringing the aforementioned components in physical proximity such that the at least one binding agent may bind to the analyte if present in the sample. It will be understood that binding of the binding agent to the analyte may require time and applying suitable conditions. The skilled person is well aware of what time is required for achieving binding and what conditions need to be applied. For example, the sample and the binding agent may be dissolved or mixed with buffers adjusting salt concentrations and/or pH value. It will be understood that suitable buffers and other auxiliary components which may be applied depend on the binding agents to be used and the chemical nature of the analyte to be determined.

As a result of the specific binding of the analyte to the at least one binding agent, said at least one binding agent is altered in its structure, e.g., by a conformational change or other molecular movement, such that the position of the at least one magnetic label becomes located closer (positive mode) or farther away (negative mode) in respect to the sensitivity field of the magnetic tunnel junction. The change in the location of the at least one magnetic label will cause an altered signal on the magnetic tunnel junction that can be detected by a detector.

In step (b) of the method of the present invention, an altered signal generated by the magnetic tunnel junction upon analyte binding to the at least one binding agent comprising the at least one magnetic label is measured.

The measuring may also encompass applying conditions such as external trigger which may be required for the detection of an altered signal elicited by the magnetic tunnel junction. In some cases, e.g., if supramagnetic magnetic labels are used, an external magnetic field may be applied. Thus, said altered signal is, preferably, induced via the at least one magnetic label by application of an external magnetic field as external trigger.

In step ( c) of the method of the invention, the analyte is determined based on the altered signal which is generated by the magnetic tunnel junction.

The determination as referred to in this context encompasses identifying the presence, absence and/or amount of magnetic labels. It will be understood that the magnetic label(s) upon binding of the analyte to the binding agent typically generate an altered signal that can be measured by the magnetic tunnel junction. The signal strength and/or duration is, typically, indicative for the amount of the magnetic label molecules present in functional proximity to the magnetic tunnel junction and, thus, also for the amount of analyte specifically bound to the at least one binding agent. Preferably, said measuring the altered signal generated by the magnetic tunnel junction upon analyte binding, thus, comprises measuring the strength of the generated altered signal and/or its duration. More preferably, said altered signal can be detected for a predetermined characteristic time period.

In the method of the present invention, different binding agents may be used for determining different analytes. In such a case, it will be understood that once such different analytes have been bound by the individual binding agents which are capable of specifically binding said different analytes, each species of binding agents must possess a different magnetic label that is capable of generating an altered signal on the magnetic tunnel junction that differs in at least one signal characteristic.

In a particular embodiment of the method of the present invention, said sensor element comprises a solid support which is a well or predetermined subdivided detection area. Preferably, each well or predetermined subdivided detection area has immobilized on it a predefined amount of binding agents. Said method is particularly useful for determining the amount of said analyte. Typically, said amount is determined by counting the wells or predetermined subdivided detection areas in which a complex has been formed. According to this particular embodiment, the presence or absence or the amount of analyte is detected for each well or predetermined subdivided area. Afterwards, the number of positive wells or predetermined subdivided areas shall be determined. A positive well or predetermined subdivided area shall be one which exhibits characteristic altered signal resulting from an analyte that is specifically bound by the binding agent as described elsewhere herein (positive or negative mode). Based on the positive wells or predetermined subdivided areas and the predefined amount of binding agents immobilized on each of said wells or predetermined subdivided areas, the amount of analyte can be determined, e.g., by calculations. For example, if each well or predetermined subdivided area contains ideally one binding agent, the presence of one molecule of analyte in a sample shall generate one altered signal. Thus, if the presence of an altered signal in a well or on a predetermined subdivided area is determined, this presence reflects the presence of analyte present in the sample. This technique is also called "digital" detection.

In the method of the present invention, in particular, for the purpose of digital detection, said sample is contacted with at least 100 sensor elements, at least 300 sensor elements, at least 500 sensor element, at least 1,000 sensor element, at least 5,000 sensor elements, at least 10,000 sensor element, at least 50,000 sensor elements or at least 100,000 sensor elements. More preferably, said determining in such as case is determining the amount of said analyte and, more preferably, said determining the amount of said analyte comprises counting the individual measured altered signals generated by the magnetic tunnel junctions and subjecting the counts to statistical analysis.

Advantageously, it has been found in accordance with the present invention that using a binding agent that upon specific analyte binding generates an altered signal of a magnetic tunnel junction can be used for determining an analyte present in a sample. Even more, a binding agent such as an aptamer which may change its structure as a result of analyte binding and which may thereby alter the position of a magnetic label víz-a-víz the magnetic tunnel junction may be particularly well suited for generating an altered signal. In the method of the present invention a kinetic measurement of specific binding and dissolution can also be determined rather than a single binding events. This allows for dynamic real-time measurements and may even make washing steps and other treatments unnecessary. Depending on the nature of the detector, it is possible to essentially singularize the binding events. In such a digital format, the number of altered signals received by the individual detectors may be used to count the analyte molecules present in the sample.

Thanks to the present invention, determination of an anaylte in a sample with an improved sensitivity and specificity is possible. Moreover, even single molecule events may be determined.

Described herein is a device for determining an analyte suspected to be present in a sample comprising at least one sensor element comprising:
(i) at least one binding agent which is capable of specifically binding to the analyte and which comprises at least one magnetic label; and in functional proximity thereto
(ii) a magnetic tunnel junction generating a signal which is altered upon binding of the analyte to the binding agent.

Typically, said device further comprises:
(iii) a detector unit capable of measuring the altered signal generated by the magnetic tunnel junction of the sensor element upon analyte binding to the at least one binding agent; and
(iv) an evaluation unit capable of determining the analyte based on the signal which is generated by the magnetic tunnel junction.

The term "device" as used herein relates to a system comprising the aforementioned components operatively linked to each other as to allow the determination of the analyte according to the method of the invention. The sensor element in accordance with the present invention may be located in a reaction zone comprised by the device. The reaction zone may either allow directly for sample application or it may be connected to a loading zone where the sample is applied. In the latter case, the sample can be actively or passively transported via the connection between the loading zone and the reaction zone to the reaction zone. Moreover, the reaction zone shall be also connected to a detector unit. Suitable detectors and detection techniques are described elsewhere herein in detail. The connection between reaction zone and detector shall be such that the detector can detect the altered signals from the magnetic tunnel junctions.

The present invention, in general, contemplates the use of the device described herein for determining an analyte suspected to be present in a sample in said sample.

Described herein is an aptamer which is capable of specifically binding to an analyte and which comprises at least one magnetic label, said aptamer being capable of altering its structure upon binding of the analyte such that the position of the at least one magnetic label is altered.

Typically, the aptamer referred to herein is a nucleic acid aptamer as specified elsewhere herein in more detail. Nucleic acid aptamers, typically, consist of DNA or RNA. They are, preferably, between 20 and 75 bases in length and have a three-dimensional structure which allow for specific binding of a target molecule such as an analyte referred to herein. More typically, said aptamer consists of at least 25 contiguous bases in length, preferably, between 25 and 75 contiguous bases in length, between 25 and 50 contiguous bases in length or, more preferably, between 25 and 35 contiguous bases in length.

Aptamers with specific binding properties and/or other properties can be generated by various techniques well known in the art including the SELEX (systematic evolution of ligands by exponential enrichment) technology or X-aptamer selection technology (see, e.g., Kaur 2019, Nanoscale Advances. 1 (6): 2123-2138 or Lokesh 2017, in Bindewald E, Shapiro BA (eds.). RNA Nanostructures. Methods in Molecular Biology. 1632. Springer New York. pp. 151-174). The aforementioned technologies may be used to generate aptamer libraries that can be used for identifying aptamer species having the desired binding as well as other properties. In particular, the method for identifying an aptamer described herein below or in the accompanying Examples may be applied.

Moreover, the aptamer, typically comprises at least one magnetic label is selected from the group consisting of: iron-platinum nanoparticles, iron nanoparticles, nickel nanoparticles, and cobalt nanoparticles.

Further, the aforementioned aptamer may comprise, typically, an adapter for immobilization. Said adapter is, more typically, LNA, L-DNA or L-LNA.

The present description also relates to a method for identifying an aptamer as defined herein before comprising the steps of:
a) providing an aptamer library;
b) identifying a plurality of aptamer candidates by an iterative selection-amplification process which are capable of specifically binding to an analyte of interest;
c) synthesizing said aptamer candidates in an array format such that each aptamer candidate is capable of regulating the signal elicited by a sensor element, preferably, a magnetic tunnel junction, linked to the aptamer candidate;
d) attaching at least one magnetic label to the aptamer candidate on the array;
e) contacting the array with the analyte of interest;
f) determining the response curve of the sensor elements based on the elicited signals; and
g) identifying the aptamer as defined herein above based on an evaluation of the individual response curves.

Preferably, steps e) and f) are iteratively repeated at least once, wherein the incubation time and conditions are altered for each iteration.

The aforementioned aptamer has been, preferably, obtained by the aforementioned method for identifying an aptamer.

Described herein is a kit for determining an analyte suspected to be present in a sample comprising the device or the aptamer described herein.

The term "kit" as used herein refers to a collection of components required for carrying out the method of the present invention including the device described above.. Typically, the components of the kit are provided in separate containers or within a single container. The container also typically comprises instructions for carrying out the method of the present invention. These instructions may be in the form of a manual or may be provided by a computer program code which is capable of carrying out or supports the determination of the analyte referred to in the methods of the present invention when implemented on a computer or a data processing device. The computer program code may be provided on a data storage medium or device such as an optical storage medium (e.g., a Compact Disc) or directly on a computer or data processing device or may be provided in a download format such as a link to an accessible server or cloud. Moreover, the kit may, usually, comprise analyte solutions with standardized amounts for calibration or validation or other reference amounts. The kit may also comprise further components which are necessary for carrying out the method of the invention such as washing solutions, solvents, and/or reagents required for detection of the detectable label. Further, it may comprise the device described above either in parts or in its entirety.

### EXAMPLES

The Examples shall merely illustrate the invention. They shall, whatsoever, not be construed as limiting the scope thereof.

### Example: Identifying an aptamer that specifically binds to an anaylte and changes its structure upon specific binding

An aptamer that specifically binds to an anaylte and changes its structure upon specific binding ("flipmer") will be identified by carrying out the following steps:
1. Provide a chip with magnetic tunnel junctions;
2. Provide a flipmer pair of choice, each carrying one or more magnetic lables, e.g., FePt-nanoparticles, which carries a linker with a specific and complementary L-LNA sequence;
3. Provide a specific immobilization L-LNA strand that carries, e.g., a linker moiety like alkine, so that it can be covalently linked to the chip surface via click-chemistry under copper induced AFM catalysis in such a way that only one or two or three flipmers are located in very close proximity to a single tunnel junction;
4. Assemble the assay platform by coating the chip with the L-LNA immobilization strands, then add the flipmers of choice for the assay or multiplex assay;
5. Read/record the blank signal levels;
6. Incubate with serum sample (eventually dilute before) and record the signals;
7. After some time enough positive signals will have been generated to allow the algorithm to calculate the concentration of the target in the sample.
8. After the assay, the chip may be regenerated by harsh washing off all components except the immobilization L-LNA.

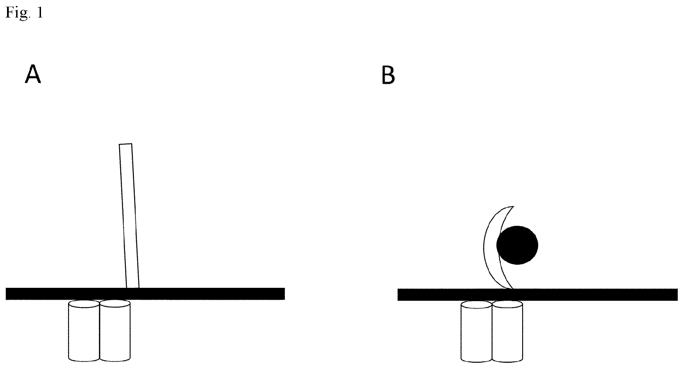

## Claims

1. A method for determining an analyte suspected to be present in a sample comprising:
(a) contacting said sample with at least one sensor element comprising:
(i) at least one binding agent which is capable of specifically binding to the analyte and which comprises at least one magnetic label; and in functional proximity thereto
(ii) a magnetic tunnel junction generating a signal which is altered upon binding of the analyte to the binding agent
for a time and under conditions which allow for specific binding of the analyte suspected to be present in the sample to the at least one binding agent;
(b) measuring an altered signal generated by the magnetic tunnel junction upon analyte binding to the at least one binding agent comprising the at least one magnetic label; and
(c) determining the analyte based on the altered signal which is generated by the magnetic tunnel junction;
wherein said at least one binding agent is an aptamer or fragment thereof, wherein said fragment refers to any fragment of said aptamer that is still capable of specifically binding to the analyte, wherein said at least one binding agent is altered in its structure upon binding to the analyte such that the position of the at least one magnetic label becomes located closer (positive mode) or farther away (negative mode) in respect to the sensitivity field of the magnetic tunnel junction.

2. The method of claim 1, wherein said measuring the altered signal generated by the magnetic tunnel junction upon analyte binding to the at least one binding agent comprises measuring the strength of the generated signal and/or its duration.

3. The method of claim 2, wherein said measuring comprises measuring a change in the altered signal over time and evaluating said change, preferably, by a computer-implemented validation algorithm.

4. The method of any one of claims 1 to 3, wherein said altered signal is induced via the at least one magnetic label by application of an external magnetic field as external trigger.

5. The method of any one of claims 1 to 4, wherein said analyte is a protein, peptide, virus, bacterial cell or small molecule.

6. The method of any one of claims 1 to 5, wherein said determining an analyte comprises determining the presence or absence of said analyte.

7. The method of any one of claims 1 to 6, wherein said sample is contacted with at least 100 sensor elements, at least 300 sensor element, at least 1,000 sensor element, at least 5,000 sensor elements, at least 10,000 sensor element, at least 50,000 sensor elements or at least 100,000 sensor elements.

8. The method of claim 7, wherein said determining an analyte comprises determining the amount of said analyte.

9. The method of claim 8, wherein said determining the amount of said analyte comprises counting the individual measured altered signals generated by the magnetic tunnel junctions and subjecting the counts to statistical analysis.

10. The method of any one of claims 1 to 9, wherein said at least one magnetic label is selected from the group consisting of: iron-platinum nanoparticles, iron nanoparticles, nickel nanoparticles, and cobalt nanoparticles.

11. The method of any one of claims 1 to 10, wherein said sample is a biological sample, preferably, a body fluid or biopsy sample.

## Patentansprüche

1. Verfahren zum Bestimmen eines Analyten, von dem vermutet wird, dass er in einer Probe vorhanden ist, umfassend:
(a) Inkontaktbringen der Probe mit mindestens einem Sensorelement, umfassend:
(i) mindestens ein Bindemittel, das in der Lage ist, spezifisch an den Analyten zu binden, und das mindestens eine magnetische Markierung umfasst; und in funktioneller Nähe dazu
(ii) einen magnetischen Tunnelübergang, der ein Signal erzeugt, das bei Bindung des Analyten an das Bindemittel verändert wird,
für eine Zeit und unter Bedingungen, die eine spezifische Bindung des Analyten, von dem vermutet wird, dass er in der Probe vorhanden ist, an das mindestens eine Bindemittel ermöglichen;
(b) Messen eines veränderten Signals, das durch den magnetischen Tunnelübergang bei Bindung des Analyten an das mindestens eine Bindemittel, das die mindestens eine magnetische Markierung umfasst, erzeugt wird; und
(c) Bestimmen des Analyten basierend auf dem veränderten Signal, das durch den magnetischen Tunnelübergang erzeugt wird;
wobei das mindestens eine Bindemittel ein Aptamer oder ein Fragment davon ist, wobei sich das Fragment auf ein beliebiges Fragment des Aptamers bezieht, das immer noch in der Lage ist, spezifisch an den Analyten zu binden, wobei das mindestens eine Bindemittel bei Bindung an den Analyten seine Struktur wechselt, sodass die Position der mindestens einen magnetischen Markierung in Bezug auf das Empfindlichkeitsfeld des magnetischen Tunnelübergangs näher (positiver Modus) oder weiter entfernt (negativer Modus) angeordnet wird.

2. Verfahren nach Anspruch 1, wobei das Messen des veränderten Signals, das durch den magnetischen Tunnelübergang bei Bindung des Analyten an das mindestens eine Bindemittel erzeugt wird, das Messen der Stärke des erzeugten Signals und/oder seiner Dauer umfasst.

3. Verfahren nach Anspruch 2, wobei das Messen das Messen einer Änderung des veränderten Signals über die Zeit und das Bewerten der Änderung, vorzugsweise durch einen computerimplementierten Validierungsalgorithmus, umfasst.

4. Verfahren nach einem der Ansprüche 1 bis 3, wobei das veränderte Signal über die mindestens eine magnetische Markierung durch Anlegen eines externen Magnetfelds als externen Auslöser induziert wird.

5. Verfahren nach einem der Ansprüche 1 bis 4, wobei der Analyt ein Protein, Peptid, Virus, eine Bakterienzelle oder ein kleines Molekül ist.

6. Verfahren nach einem der Ansprüche 1 bis 5, wobei das Bestimmen eines Analyten das Bestimmen des Vorhandenseins oder der Abwesenheit des Analyten umfasst.

7. Verfahren nach einem der Ansprüche 1 bis 6, wobei die Probe mit mindestens 100 Sensorelementen, mindestens 300 Sensorelementen, mindestens 1.000 Sensorelementen, mindestens 5.000 Sensorelementen, mindestens 10.000 Sensorelementen, mindestens 50.000 Sensorelementen oder mindestens 100.000 Sensorelementen in Kontakt gebracht wird.

8. Verfahren nach Anspruch 7, wobei das Bestimmen eines Analyten das Bestimmen der Menge des Analyten umfasst.

9. Verfahren nach Anspruch 8, wobei das Bestimmen der Menge des Analyten das Zählen der einzelnen gemessenen veränderten Signale, die durch die magnetischen Tunnelübergänge erzeugt werden, und das Unterwerfen der Zählungen einer statistischen Analyse umfasst.

10. Verfahren nach einem der Ansprüche 1 bis 9, wobei die mindestens eine magnetische Markierung ausgewählt ist aus der Gruppe, bestehend aus: Eisen-Platin-Nanopartikeln, Eisen-Nanopartikeln, Nickel-Nanopartikeln und Kobalt-Nanopartikeln.

11. Verfahren nach einem der Ansprüche 1 bis 10, wobei die Probe eine biologische Probe, vorzugsweise eine Körperflüssigkeits- oder Biopsieprobe, ist.

## Revendications

1. Procédé de détermination d'un analyte suspecté d'être présent dans un échantillon comprenant :
(a) la mise en contact dudit échantillon avec au moins un élément capteur comprenant :
(i) au moins un agent de liaison qui est capable de se lier spécifiquement à l'analyte et qui comprend au moins un marqueur magnétique ; et à proximité fonctionnelle de celui-ci
(ii) une jonction tunnel magnétique générant un signal qui est modifié lors de la liaison de l'analyte à l'agent de liaison
pendant une durée et dans des conditions qui permettent une liaison spécifique de l'analyte suspecté d'être présent dans l'échantillon à l'au moins un agent de liaison ;
(b) la mesure d'un signal modifié généré par la jonction tunnel magnétique lors de la liaison de l'analyte à l'au moins un agent de liaison comprenant l'au moins un marqueur magnétique ; et
(c) la détermination de l'analyte sur la base du signal modifié qui est généré par la jonction tunnel magnétique ;
dans lequel ledit au moins un agent de liaison est un apatmère ou un fragment de celui-ci, dans lequel ledit fragment fait référence à un fragment quelconque dudit aptamère qui est encore capable de se lier spécifiquement à l'analyte, dans lequel ledit au moins un agent de liaison est modifié au niveau de sa structure lors de la liaison à l'analyte de sorte que la position de l'au moins un marqueur magnétique se rapproche (mode positif) ou s'éloigne (mode négatif) par rapport au champ de sensibilité de la jonction tunnel magnétique.

2. Procédé selon la revendication 1, dans lequel ladite mesure du signal modifié généré par la jonction tunnel magnétique lors de la liaison de l'analyte à l'au moins un agent de liaison comprend la mesure de l'intensité du signal généré et/ou de sa durée.

3. Procédé selon la revendication 2, dans lequel ladite mesure comprend la mesure d'une variation dans le signal modifié en fonction du temps et l'évaluation de ladite variation, de préférence, par un algorithme de validation mis en œuvre par ordinateur.

4. Procédé selon l'une quelconque des revendications 1 à 3, dans lequel ledit signal modifié est induit par l'intermédiaire de l'au moins un marqueur magnétique par application d'un champ magnétique externe en tant que déclencheur externe.

5. Procédé selon l'une quelconque des revendications 1 à 4, dans lequel ledit analyte est une protéine, un peptide, un virus, une cellule bactérienne ou une petite molécule.

6. Procédé selon l'une quelconque des revendications 1 à 5, dans lequel ladite détermination d'un analyte comprend la détermination de la présence ou de l'absence dudit analyte.

7. Procédé selon l'une quelconque des revendications 1 à 6, dans lequel ledit échantillon est mis en contact avec au moins 100 éléments capteurs, au moins 300 éléments capteurs, au moins 1 000 éléments capteurs, au moins 5 000 éléments capteurs, au moins 10 000 éléments capteurs, au moins 50 000 éléments capteurs ou au moins 100 000 éléments capteurs.

8. Procédé selon la revendication 7, dans lequel ladite détermination d'un analyte comprend la détermination de la quantité dudit analyte.

9. Procédé selon la revendication 8, dans lequel ladite détermination de la quantité dudit analyte comprend le comptage des signaux individuels modifiés mesurés générés par les jonctions tunnel magnétiques et la soumission des comptages à une analyse statistique.

10. Procédé selon l'une quelconque des revendications 1 à 9, dans lequel ledit au moins un marqueur magnétique est choisi dans le groupe constitué par : des nanoparticules de fer-platine, des nanoparticules de fer, des nanoparticules de nickel et des nanoparticules de cobalt.

11. Procédé selon l'une quelconque des revendications 1 à 10, dans lequel ledit échantillon est un échantillon biologique, de préférence un échantillon de fluide corporel ou de biopsie.
